# EUROPÄISCHE PATENTANMELDUNG

(11) **EP 1 736 535 A1**
(43) Veröffentlichungstag der Anmeldung: **27.12.2006**
(21) Anmeldenummer: 06012574.7
(22) Anmeldetag: 20.06.2006
(51) Int. Cl.: C12M 1/107

(54) **Verfahren zum Betreiben einer Feststofffermenteranlage sowie Vorrichtung hierzu**

(30) Priorität: 22.06.2005 DE 102005029306
(71) Anmelder: Firma Joachim Kausch, 70599 Stuttgart (DE); Kreidl, Alfred, 72160 Horb-Dießen (DE)
(72) Erfinder: Kausch, Joachim, 70599 Stuttgart (DE); Kreidl, Alfred, 72160 Horb-Dießen (DE)
(74) Vertreter: Langöhrig, Angelika Beate

(57) **Zusammenfassung**

Die Erfindung betrifft ein Verfahren zum Betreiben einer Feststofffermenteranlage, umfassend mindestens zwei diskontinuierlich und zeitlich zueinander versetzt betreibbare Feststofffermenter, wobei die Fermenter von oben nach unten mit einem Perkolat durchströmt werden und das abfließende Perkolat aller Fermenter zusammengeführt und erneut den Fermentern zugeführt wird, wobei ein solcher Perkolatstrom je Fermenter eingestellt wird, dass in dem Fermenter entstehende Säuren mit dem Perkolatstrom insoweit ausgeschwemmt werden, dass keine Versauerung der Fermenter eintritt und der Perkolatstrom eine solche Beladung mit Mikroorganismen aufweist, dass er zum Starten der Fermentation in einem neu angesetzten Fermenter einsetzbar ist.

## Beschreibung

Die vorliegende Erfindung betrifft ein Verfahren zum Betreiben einer Feststofffermenteranlage, umfassend mindestens zwei diskontinuierlich und zeitlich zueinander versetzt betreibbare Feststofffermenter, wobei die Fermenter von oben nach unten mit einem Perkolat durchströmt werden und das abfließende Perkolat aller Fermenter zusammengeführt und erneut den Fermentern zugeführt wird.

Bei dem vorliegenden Verfahren handelt es sich um die anaerobe Fermentation von Biomasse, einer Alternative zu der bereits verbreitet eingesetzten Kompostierung. Dabei wird Biomasse in einem anaeroben Milieu von geeigneten Bakterien unter Bildung von Biogas, insbesondere Methan, zersetzt, wobei sowohl einstufige als auch zweistufige Verfahren bekannt sind. Unter zeitlich zueinander versetzt, soll verstanden werden, dass das Anfahren der einzelnen Batchprozesse nicht zeitgleich erfolgt. Die Fermenter befinden sich dadurch in verschiedenen Stadien des Vergärprozesses.

Es sind in der Landwirtschaft vor allem Flüssigfermenter sowie in der Abfallwirtschaft entweder Flüssigfermenter oder Fermenter für pumpfähig gemachte Bioabfälle im Einsatz. Hierbei wird die Biomasse vor der Vergärung zur Erzeugung eines homogenen, pump- und rührfähigen Substrats aufbereitet, wobei der mit der Aufbereitung verbundene hohe Aufwand nachteilig zu beurteilen ist. Darüber hinaus sind derartige Verfahren der Nassfermentation vergleichsweise störanfällig insbesondere im Hinblick, auf Fremdstoffe. Daher ist in letzter Zeit zunehmend die Trocken- oder Feststofffermentation in das Interesse gerückt. Diese ist insbesondere bei landwirtschaftlichen Betrieben ohne Viehhaltung, die nachwachsende Rohstoffe vergären wollen, von Interesse. Hierbei werden vielfach sogenannte "berieselte Boxenfermenter" eingesetzt, wie sie beispielsweise in der EP 1 428 868 A1 beschrieben sind, bei denen eine Fermenterzelle in Form eines Boxenfermenters von unten nach oben mit Gas durchströmt wird, um die Vergärung zu fördern. Problematisch ist hierbei die unsichere Betriebsweise, da eine Verstopfungsgefahr der Gasdüsen besteht.

Des Weiteren ist beispielsweise aus der EP 1 301 583 B1 ein Bioreaktor zur Methanisierung von Biomasse und eine Biogasanlage bekannt, die ebenfalls auf dem Prinzip des Boxenfermenters beruht, wobei hierbei im Boden eine Heizeinrichtung eingebracht ist, um den Fermenter auf die notwendige für die Mikroorganismen erforderliche Temperatur aufzuheizen. Nachteilig ist hierbei, dass aufgrund des schlechten Wärmeübergangs eine vollständige Durchheizung des Biomaterials nur unbefriedigend bzw. mit hohen Verlusten möglich ist.

Unter Boxenfermentern werden hierbei garagenartige Boxen verstanden, die mit Biomasse befüllt und mit Prozesswasser, dem sogenannten "Perkolat", berieselt werden. Das Perkolat wird am Fuß der Boxen erfasst, im Kreis geführt und wieder verrieselt.

Zum Starten der Fermentation in einem neu anzusetzenden Fermenter wird diesem ein sogenannter Gärrest aus bereits angefaultem Material als Starterkultur zugesetzt und weiterhin frisches Material eingefüllt. Der Anteil des Gärrestes liegt bezogen auf das Gewicht in der Regel über dem Anteil an frischem Material, insbesondere zwischen 30% und 50%, teilweise sogar bei über 80%.

Nachteilig ist hierbei, dass aufgrund des hohen Anteils an Gärrest die Fermenter entsprechend groß dimensioniert werden müssen, um für die Gasgewinnung einen ausreichenden Anteil frischer Biomasse zu umfassen. Als zu vergärende Biomasse kommt insbesondere Biomüll, aber auch Maissilage sowie sämtliche anderen nachwachsenden Rohstoffe, etc. in Frage. Dabei muss der Gärrest umso größer sein, je energiehaltiger die einzubringende Biomasse ist.

Die Vermischung von Gärrest und frischem Material vor dem Einbringen in den Fermenter stellt einen zusätzlichen Arbeitsschritt dar, der darüber hinaus aus Sicht des Emissionsschutzes bei Mischen des Gärrests mit dem Biomüll im Freien kritisch zu beurteilen ist.

Es ist daher Aufgabe der Erfindung, ein Verfahren sowie eine Vorrichtung hierzu bereitzustellen, mit dem bei gleichem Anlagenvolumen größere Mengen an Biomasse vergoren werden können, wobei auf der anderen Seite eine gute Methanbildung eingestellt werden kann.

Die Erfindung löst diese Aufgabe durch ein Verfahren und eine Vorrichtung, wobei ein solcher Perkolatstrom je Fermenter eingestellt wird, dass in dem Fermenter entstehende Säuren mit dem Perkolatstrom zumindest teilweise ausgeschwemmt werden und der Perkolatstrom eine solche Beladung mit Mikroorganismen aufweist, dass er zum Starten der Fermentation in einem neu angesetzten Fermenter einsetzbar ist. Die Ausschwemmung erfolgt dabei derart, dass keine Versauerung der Fermenter auftritt. Unter Versauerung ist dabei zu verstehen, dass aufgrund der vorliegenden Säuren eine weitere Vergärung nicht stattfindet.

Ein entsprechendes Verfahren besitzt den Vorteil, dass durch die Ausschwemmung der Säuren aus der Biomasse erreicht wird, dass ein Fermenter insbesondere in der Anfahrphase nicht in die saure Phase übergeht. So erfolgt beim Abbau von Biomasse zunächst eine Glykolyse, wobei durch die an der Methanbildung beteiligten Mikroorganismen zumindest teilweise organische Säuren gebildet werden. Diese Säurebildung ist kritisch zu betrachten. Unter den Startbedingungen im Fermenter ist z. B. die bakterielle Verdoppelungszeit für Propionsäurebildner erheblich kürzer als für Essesäurebildner und für acetoclasische, also Essigsäure abbauende Methanbildner. Während Essigsäure als die beste methanogene Substanz angesehen wird, hemmt Propionsäure in höherer Konzentration die Essigsäurebildung und die Essigsäureverwertung. Darüber hinaus wirkt die ebenfalls entstehende Milchsäure bakterizid, so dass hierdurch die weitere Vermehrung von Mikroorganismen und damit die Essigsäure- und Methanbildung weiter gehemmt wird. Dies stellt eine kritische Phase zu Beginn der Fermentation dar. Es ist daher vorteilhaft, wenn der Perkolatstrom so eingestellt wird, dass organische Säuren, insbesondere Propion- und Milchsäure, mit dem Perkolat ausgeschwemmt wird. Der Perkolatstrom ist daher so einzustellen, dass die Säuren insbesondere im Anfangsstadion der Fermentation ausgeschwemmt werden, da durch den entstehenden niedrigen pH eine Milchsäurebildung weiter gefördert wird. Es müssen daher die Säuren in einem solchen Maße ausgeschwemmt werden, dass eine Hemmung der Fermentation und der Organismenvermehrung nicht eintritt. Auf diese Weise können verstärkt Essigsäure bildende Mikroorganismen sowie Essigsäure abbauende Mikroorganismen im Fermenter gebildet werden. Dabei kann vorgesehen sein, dass bei einer starken Säurebeladung des Perkolats das Perkolat verstärkt oder ausschließlich auf solche Feststofffermenter verteilt wird, die bereits länger im Fermentationsprozess befindlich sind und so die entstehenden Säuren aufgrund ihrer bereits höheren Mikroorganismenbeladung verarbeiten können. Das Perkolat dient dabei als Energie- und/oder Nährstoffeintrag in einen solchen Fermenter. Auf diese Weise kann das Perkolat bei zu starker Säurebeladung durch einen frisch angesetzten Fermenter durch den mindestens einen weiteren, bereits länger laufenden Fermenter aufbereitet, also die Säuren abgebaut werden.

Darüber hinaus wird mit dem Perkolat, insbesondere mit der Perkolation durch den mindestens einen bereits länger laufenden Fermenter, eine Vielzahl von Mikroorganismen mit ausgeschwemmt, die mit dem Perkolat in einen frisch anzusetzenden Fermenter eingetragen werden und hier als Starterkultur dienen. Die Verwendung eines Gärrests kann so in vielen Fällen vollständig vermieden werden. In jedem Fall kann jedoch die erforderliche Rückmischung von Gärrest deutlich unter dem im Stand der Technik einzusetzenden Gärrest gehalten werden.

Besonders bevorzugt kann dabei vorgesehen sein, dass ein Perkolatbehälter vorgesehen ist, der als Perkolatzwischenspeicher und Regenerationsfermenter dient, und der als zusätzlicher insbesondere kontinuierlicher Fermenter betrieben werden kann, der ebenfalls zur Erzeugung von Biogas beiträgt. Dabei können die im Perkolat enthaltenen Mikroorganismen die ebenfalls im Perkolat enthaltenen Säuren zu Biogas umsetzen, so dass vorgesehen sein kann, dass auch der Perkolatbehälter zur Biogasgewinnung und zum Säureabbau beiträgt. Das durch die verschiedenen Fermenter erzeugte Biogas kann dabei in einzelnen oder einem gemeinsamen Biogasreservoir aufgefangen werden, aus dem Biogas zur weiteren Verwertung entnommen werden kann und der Energiegewinnung dient.

Alternativ oder zusätzlich zum Perkolatbehälter kann auch ein weiterer Fermenter, der mit Feststoff befüllt ist, ebenfalls als Regenerationsfermenter betrieben werden. Der Fermenter wird dabei im Gegensatz zu den übrigen Fermentern nicht im Batchbetrieb, sondern kontinuierlich betrieben. Als Füllgut kommen dabei alle sich als Siedlungsstruktur für die Organismen eignenden Materialien in Frage, die langfristig stabil und flüssigkeitsdurchlässig sind, wie z. B. Holzstrukturen, z. B. Hackschnitzel, Kunststofffüllkörper, Blähton, etc. Dieser Fermenter dient dabei zur Verringerung des Säuregehalts im Perkolat und ebenfalls zur Methanerzeugung.

Des Weiteren kann es besonders vorteilhaft vorgesehen sein, dass der Perkolatbehälter und/oder das Perkolatleitungssystem beheizt wird. Durch den vorgesehenen verhältnismäßig großen Perkolatstrom, der durch die Biomasse sickert, kann erreicht werden, dass auf diese Weise die Biomasse über das Perkolat beheizt wird. Dabei arbeiten die der Methanisierung dienenden Mikroorganismen vorzugsweise bei einer Temperatur von > 30°. Eine Beheizung der Flüssigkeit im Perkolatbehälter oder in den Leitungen lässt sich dabei erheblich effizienter gestalten als die Beheizung der Biomasse selbst und eine bessere Durchheizung kann erzielt werden, da der Wärmeübergang im Perkolat gegenüber dem der Biomasse erheblich besser ist. Durch die möglichst gleichmäßige Verteilung des Perkolats über die gesamte Biomasse wird darüber hinaus eine gleichmäßige Erwärmung der Biomasse erreicht.

Erfindungsgemäß kann vorgesehen sein, dass neben der Verwendung des Perkolats als Starterkultur als zusätzliche Starterkultur ein Gärrest im Bereich von 0 bis 30 Gew.-%, insbesondere von 0 bis 20 Gew.-%, insbesondere von 0 bis 10 Gew.-% und vorzugsweise von 0 Gew.-% bezogen auf das Gesamtgewicht der Biomasse in einem der Fermenter zugesetzt wird.

Das Verfahren kann grundsätzlich bis zu einem minimalen pH-Wert im abgezogenen Perkolat des neu angesetzten Fermenters bis ca. 2,5 gefahren werden.

Des Weiteren betrifft die Erfindung eine Feststofffermenteranlage, insbesondere zur Durchführung des Verfahrens umfassend mindestens zwei Feststofffermenter, die über Perkolatzu- und -ableitungen mit einem Regenerationsfermenter verbunden sind. Der Regenerationsfermenter dient ebenfalls als Fermenter zur Erzeugung von Biogas. Er kann insbesondere ein kontinuierlich betriebener Flüssig- oder Feststofffermenter sein.

Insbesondere kann die Anlage drei oder vier Feststofffermenter oder ein Vielfaches davon, die im Batchbetrieb betrieben werden, umfassen, wobei die bevorzugte Anzahl u. a. von der Dauer der Fermentation abhängt. So kann insbesondere vorgesehen sein, dass zur vollständigen Fermentation der Biomasse in einem Fermenter im Batchverfahren ca. 4 Wochen benötigt werden. In diesem Fall ist es vorteilhaft, vier Feststofffermenter oder ein Vielfaches hiervon einzusetzen, wobei jede Woche einer oder mehrere der Fermenter neu angesetzt wird. Bei anderen Zeiträumen, die zur vollständigen Umsetzung der Biomasse benötigt werden, können andere Fermenterzahlen eingesetzt werden. Als Fermenter können hierbei insbesondere Boxenfermenter oder auch runde Fermenterformen eingesetzt werden, wobei eine Heizvorrichtung am Perkolatbehälter und/oder am Rohrleitungssystem vorgesehen sein kann. Insbesondere weist die Feststofffermenteranlage einen Biogassammelbehälter auf, über den das Biogas dann zur weiteren Verarbeitung abgegeben wird.

Grundsätzlich kann kontinuierlich oder zu bestimmten Zeitpunkten eine Gasmessung und/oder -analyse an verschiedenen Stellen der Anlage vorgenommen werden, auf die jedoch auch verzichtet werden kann.

Die Umpumpung und Verteilung des Perkolats kann mittels einer oder mehrerer Pumpen und/oder auch über Ventile und/oder im freien Gefälle erfolgen.

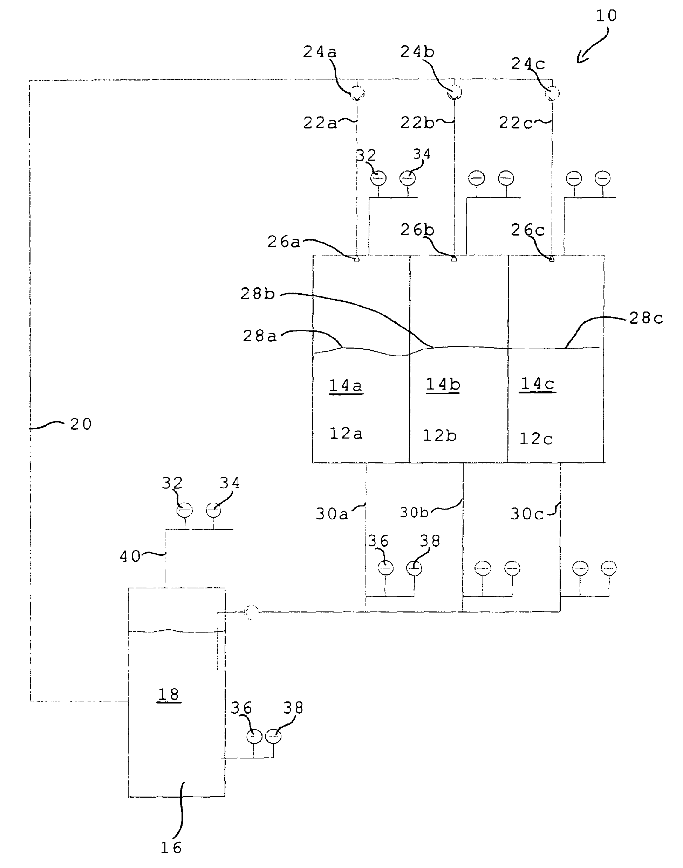

Die Erfindung soll im Folgenden anhand eines Ausführungsbeispiels näher erläutert werden. Dabei zeigt die Zeichnung in der einzigen Figur eine Feststofffermenteranlage, die in ihrer Gesamtheit mit dem Bezugszeichen 10 versehen ist. Die Feststofffermenteranlage 10 umfasst hierbei drei Feststofffermenter, die mit 12a bis 12c bezeichnet sind. Die Feststofffermenter, die eine garagenartige Form aufweisen können und von vorne ähnlich wie eine Autogarage mit Biomasse befüllt werden können, sind mit Biomasse, die jeweils 14a bis 14c gekennzeichnet ist, beladen.

Des Weiteren umfasst die Feststofffermenteranlage 10 einen Perkolatbehälter 16, in dem Perkolationswasser 18 zwischengespeichert wird, das zur Verdüsung in den Fermentationsbehältern 12a bis 12c verwendet wird, wobei das Perkolat 18 am Fuße der Fermenter 12a bis 12c aufgefangen und in den Perkolatbehälter 16 zurückgeführt wird. Die Perkolatverteilung ist hierbei durch eine Leitung 20 gekennzeichnet, über die Perkolat 18 aus dem Perkolatbehälter 16 entnommen wird und sich diese Leitung in drei Einzelleitungen 22a, 22b und 22c aufteilt, die jeweils einem Fermenter 12a bis 12c zugeordnet sind. Mittels Pumpen 24 wird hierbei das Perkolat zu Verteileinrichtungen 26a bis 26c gefördert und möglichst gleichmäßig über die Oberflächen 28a-28c der einzelnen Fermenter und der darin angeordneten Biomasse 14a bis 14c verteilt, um einen möglichst gleichmäßigen Kontakt der Biomasse 14a bis 14c mit dem Perkolat 18 sicherzustellen und eine gute Durchfeuchtung zu erreichen.

Am Boden der Fermentationsbehälter 12a bis 12c wird das Perkolat 18 dann über Leitungen 30a bis 30c wieder abgezogen. Die Leitungen 30 werden zusammengeführt und münden wieder in den Perkolatbehälter 16.

Zur weiteren Überwachung der Fermentation werden sowohl die Gasmenge über Gaszähler 32 gemessen als auch eine Gasanalyse, die mit 34 gekennzeichnet ist, erstellt. Darüber hinaus wird sowohl im Perkolatbehälter 16 als auch in den Perkolatabläufen 30a bis 30c der pH-Wert sowie die Temperatur durch Einrichtungen 36 und 38 bestimmt.

Bei Fermentationsanlagen 10 mit mehreren Feststofffermentern 12a bis 12c erfolgt eine Beladung der Fermenter mit frischer Biomasse nicht zeitgleich, sondern zeitlich zueinander versetzt, beispielsweise derart, dass jede Woche ein Fermenter 12a bis 12c angesetzt wird, wobei im vorliegenden Fall es besonders vorteilhaft sein kann, wenn die vollständige Fermentation drei Wochen dauert, so dass stets ein Fermenter sich in der ersten Woche, ein Fermenter sich in der zweiten Woche und ein Fermenter sich in der dritten und letzten Woche befindet. Wird ein Fermenter neu angesetzt, d. h. neu mit Biomasse befüllt, so muss zum Starten der Vergärung eine Starterkultur hinzugegeben werden bzw. der Fermenter angeimpft werden. Im vorliegenden Fall soll davon ausgegangen werden, dass der Fermenter 12a neu mit Biomasse 14a befüllt ist. Die beiden anderen Fermenter 12b und 12c befinden sich in den Wochen zwei und drei, d. h. im Bereich der stabil verlaufenden Vergärung. In beiden Fermentern 12b und 12c sind bereits Mikroorganismen in solcher Anzahl vorhanden, dass eine stabile Vergärung und Methanproduktion erfolgt. Über die Verdüsung von Perkolat 18 in den Fermentern 12b und 12c und den Wiederabzug von Perkolat 18 am Boden der Fermenter 12b und 12c werden Mikroorganismen mit dem Perkolat 18 ausgespült. Wird nun das Perkolat mit den Mikroorganismen über die Leitung 22a dem neu angesetzten Fermenter 12a zugeführt, so dienen diese Mikroorganismen zum Start der Vergärung im Fermenter 12a. Eine Zugabe von Gärrest, d. h. einem Rest von bereits vergorener organismenreicher Biomasse vor Neuansetzen einer Fermentation im Fermenter 12a zum Starten der Fermentation ist auf diese Weise nicht notwendig.

Beginnt nun die Fermentation im Fermenter 12a, so beginnen zunächst die Säure bildenden Organismen Säure zu produzieren. Wie bereits ausgeführt, besitzen die Säure bildenden Mikroorganismen eine erheblich kürzere Reproduktionsphase als die Säure abbauenden Mikroorganismen, die Methangas bilden. Diese benötigen hiergegen erheblich länger. Um einer Versäuerung des Fermenters 12a vorzubeugen, wird der Perkolatstrom über die Verteileinrichtung 26a so eingestellt, dass mittels des Perkolats 18, das durch die Biomasse 14a im Fermenter 12a hindurchsickert, die Säuren mit ausgetragen und im Perkolatbehälter 16 mit den Perkolatströmen aus den Fermentern 12b und 12c vermischt werden. Auf diese Weise kann die Konzentration an Säure deutlich erniedrigt werden und die Vermehrung der benötigten Organismen wird nicht durch die Versäuerung gehemmt. Besonders vorteilhaft ist hierbei, dass zum Animpfen des neu angesetzten Fermenters 12a kein Gärrest mehr notwendig ist, wobei es bei der Vormischung von neuer Biomasse und Gärrest insbesondere bei einer Anmischung im Freien zu einer starken Emissionsbelastung kommen kann.

Insbesondere wenn das Perkolat im Perkolatbehälter 16 hinsichtlich des pH-Werts zu sauer zu werden droht, kann vorgesehen sein, dass das Perkolat über einen begrenzten Zeitraum ausschließlich oder hauptsächlich den Fermentern 12b und 12c, die bereits länger laufen, zugeführt wird, da hier bereits eine stabile Mikroorganismenpopulation existiert, die derartiges Perkolat umsetzen und die darin enthaltenen Nährstoffe zu Methangas verarbeiten kann. Auf diese Weise kann eine erheblich verbesserte Regelung der Fermentation und damit auch der Methangasproduktion erreicht werden.

Schließlich wurde erfindungsgemäß festgestellt, dass auch der Perkolatbehälter 16 als Fermenter wirkt, so dass auch hier über eine Gasentnahmeleitung 40 ein Methangasstrom entnommen werden kann, der über eine Gasuhr 32 sowie eine Gasanalyseeinrichtung 34 bestimmt wird. Auf diese Weise kann neben den bereits bestehenden Fermentern 12a bis 12c auch über den Perkolattank 16 noch weiteres Methangas erzeugt werden. Der Perkolattank 16 dient darüber hinaus auch dem Säureabbau und damit der Regeneration des Perkolats.

Als Gasmessgeräte werden insbesondere solche für Methan, aber auch CO₂ und Sauerstoff eingesetzt. Es ist hierbei vorteilhaft, wenn die Werte entweder kontinuierlich oder in bestimmten Intervallen z. B. 24-stündlich abgelesen werden.

Darüber hinaus besitzt eine intensive Perkolation noch einen weiteren Vorteil. So lässt sich das Perkolat im Behälter 16 erheblich leichter aufheizen als der Biomüll 14a bis 14c in den Fermentern 12a bis 12c, da organische Substanzen lediglich einen schlechten Wärmeübergang besitzen. Auf diese Weise kann sowohl auf eine Fußbodenheizung als auf eine Vorrotte, die bisher vorgesehen ist, um die Temperatur in der Biomasse 14a bis 14c so anzuheizen, dass über den Fermentationsprozess eine ausreichende Temperatur gehalten wird, verzichtet werden. Vielmehr kann die Flüssigkeit 18 im Behälter 16 auf die erwünschte Temperatur eingestellt werden und durch die starke Perkolation in den einzelnen Fermentern 12a-12c wird diese Wärme gleichmäßig über das gesamte Volumen in die Fermenter 12a-12c eingetragen.

Ein weiterer Vorteil besteht darin, dass durch das Vermeiden des Zusatzes von Gärrest zum einen die Fermentervolumen verkleinert werden können, was in geringeren Investitionen resultiert und auf der anderen Seite Verweilzeiten bzw. der Durchsatz erhöht werden können, wodurch sich der Gasertrag erhöht.

Die vorliegende Erfindung bietet daher ein gut zu steuerndes Feststofffermentationsverfahren bzw. ein entsprechende Vorrichtung hierzu, die auch auf dem landwirtschaftlichen Sektor einfach und sicher zu handhaben ist und darüber hinaus einen guten Gasertrag ermöglicht und den Emissionsgesichtspunkten Rechnung trägt.

## Patentansprüche

1. Verfahren zum Betreiben einer Feststofffermenteranlage, umfassend mindestens zwei diskontinuierlich und zeitlich zueinander versetzt betreibbare Feststofffermenter, wobei die Fermenter von oben nach unten mit einem Perkolat durchströmt werden und das abfließende Perkolat aller Fermenter zusammengeführt und erneut den Fermentern zugeführt wird, wobei ein solcher Perkolatstrom je Fermenter eingestellt wird, dass in dem Fermenter entstehende Säuren mit dem Perkolatstrom insoweit ausgeschwemmt werden, dass keine Versauerung der Fermenter eintritt und der Perkolatstrom eine solche Beladung mit Mikroorganismen aufweist, dass er zum Starten der Fermentation in einem neu angesetzten Fermenter einsetzbar ist.

2. Verfahren nach Anspruch 1, wobei ein kontinuierlich arbeitender Regenerationsfermenter im Perkolatkreislauf betrieben wird.

3. Verfahren nach einem der Ansprüche 1 oder 2, wobei ein Perkolatbehälter vorgesehen ist zum Zwischenspeichern von Perkolat, der als Regenerationsfermenter arbeitet.

4. Verfahren nach einem der Ansprüche 1 bis 3, wobei ein Feststofffermenter als Regenerationsfermenter betreibbar ist.

5. Verfahren nach einem der vorangehenden Ansprüche, wobei das Perkolat möglichst gleichmäßig über einen Fermenterquerschnitt verteilt wird.

6. Verfahren nach einem der vorangehenden Ansprüche, wobei das Perkolat, insbesondere im Perkolatbehälter und/oder in einem Perkolatleitungssystem beheizt wird.

7. Verfahren nach einem der vorangehenden Ansprüche, wobei das Perkolat dem Energie- und/oder Nährstoffeintrag in einen oder mehrere Fermenter dient.

8. Verfahren nach einem der vorangehenden Ansprüche, wobei die Aufteilung des Perkolatstroms auf die Fermenter anhand der Beladung des Perkolats mit Säuren und Mikroorganismen geregelt wird.

9. Verfahren nach einem der vorangehenden Ansprüche, wobei beim Start einem Fermenter ein Gärrest als Starterkultur im Bereich von 0-30 Gew.-%, insbesondere von 0-20 Gew.-%, insbesondere von 0-10 Gew.-% und vorzugsweise von 0 Gew.-% zugesetzt wird.

10. Verfahren nach einem der vorangehenden Ansprüche, wobei es sich um ein methanerzeugendes Verfahren handelt.

11. Feststofffermenteranlage, insbesondere zur Durchführung des Verfahrens nach einem der vorangehenden Ansprüche, umfassend mindestens zwei Feststofffermenter (12a-c), die über Perkolatzu- (20) und -ableitungen (30) mit einem kontinuierlich betreibbaren Regenerationsfermenter (16) verbunden sind.

12. Feststofffermenteranlage nach Anspruch 11, wobei als Regenerationsfermenter ein Perkolatbehälter (16) oder ein Feststofffermenter dient.

13. Feststofffermenteranlage nach Anspruch 11 oder 12, wobei pro Woche Fermentationsdauer mindestens ein Fermenter vorgesehen ist (12a-12c).

14. Feststofffermenteranlage nach einem der Ansprüche 11 bis 13, wobei die Feststofffermenter (12a-c) Boxenfermenter oder runde Fermenter sind.

15. Feststofffermenteranlage nach einem der Ansprüche 11 bis 14, wobei der Perkolatbehälter (16) und/oder das Perkolatleitungssystem eine Heizung aufweist.
